(19) Europäisches Patentamt
European Patent Office
Office européen des brevets

(11) EP 4 741 384 A1

(12) EUROPEAN PATENT APPLICATION
published in accordance with Art. 153(4) EPC

(43) Date of publication:
13.05.2026 Bulletin 2026/20

(21) Application number: 24836087.7

(22) Date of filing: 03.07.2024

(51) International Patent Classification (IPC):
*C07D 307/68 (2006.01)* *C07B 61/00 (2006.01)*

(52) Cooperative Patent Classification (CPC):
C07B 61/00; C07D 307/68

(86) International application number:
PCT/JP2024/024148

(87) International publication number:
WO 2025/009575 (09.01.2025 Gazette 2025/02)

(84) Designated Contracting States:
AL AT BE BG CH CY CZ DE DK EE ES FI FR GB GR HR HU IE IS IT LI LT LU LV MC ME MK MT NL NO PL PT RO RS SE SI SK SM TR
Designated Extension States:
BA
Designated Validation States:
GE KH MA MD TN

(30) Priority: 04.07.2023 JP 2023110316

(71) Applicant: MITSUBISHI GAS CHEMICAL COMPANY, INC.
Chiyoda-ku
Tokyo 100-8324 (JP)

(72) Inventors:
- UMEZU Ryotaro
Tokyo 125-8601 (JP)
- IMADA Shin-ichiro
Tokyo 125-8601 (JP)
- SHINKAI Yousuke
Tokyo 125-8601 (JP)
- HARADA Hidefumi
Tokyo 125-8601 (JP)
- ISOBE Takehiko
Tokyo 125-8601 (JP)

(74) Representative: Hoffmann Eitle
Patent- und Rechtsanwälte PartmbB
Arabellastraße 30
81925 München (DE)

# (54) FURONITRILE COMPOUND PRODUCTION METHOD AND CARBONATE ESTER PRODUCTION METHOD

(57) The present invention provides a method that makes it possible to selectively obtain a desired compound with high yield while shortening reaction time in regeneration of a nitrile compound by dehydration of an amide compound, and that also suppresses the generation of by-products. This problem is solved by a furonitrile compound production method comprising a dehydration reaction for dehydration of a furamide compound, wherein the dehydration reaction has a contact step for bringing a diluent and the furamide compound in a gaseous phase into contact with a catalyst. (In formula (1), $R_1$ and n are as defined in the description.)

EP 4 741 384 A1

## Description

### TECHNICAL FIELD

The present invention relates to a method for producing a furonitrile compound and a method for producing a carbonate ester.

### BACKGROUND ART

The term carbonate ester collectively refers to compounds in which one or both of the two hydrogen atoms in carbonic acid, $CO(OH)_2$, are substituted with an alkyl group or an aryl group, and which have a structure represented by RO-C(=O)OR' (where R and R' each represent a hydrogen atom, a saturated hydrocarbon group, an unsaturated hydrocarbon group, or the like).

Carbonate esters are highly useful compounds that are used not only as additives, such as gasoline additives for improving the octane value and diesel fuel additives for decreasing particulate matter in exhaust gas, but also as alkylating agents, carbonylating agents, and solvents in the synthesis of resins and organic compounds such as polycarbonates, urethanes, pharmaceuticals, and agrochemicals. In addition, they are used as electrolytic solutions for lithium ion batteries, as raw materials for lubricating oils, and as raw materials for deoxidizing agents for preventing corrosion in boiler pipes.

As conventional methods for producing a carbonate ester, for example, methods are known that include a step of allowing water, which is generated as a by-product in a reaction between an alcohol and carbon dioxide, to react with a nitrile compound (Patent Literatures 1 to 3 and the like). In such methods for producing a carbonate ester, a step is included in which the nitrile compound is regenerated by dehydrating an amide compound generated from the water and the nitrile compound.

According to such conventional methods for producing a carbonate ester, the nitrile compound cannot necessarily be regenerated selectively in a short period of time and with high yield, and it has not been easy to efficiently produce a desired compound, the carbonate ester.

### PRIOR ART DOCUMENTS

### PATENT DOCUMENTS

Patent Literature 1: WO 2015/099053
Patent Literature 2: WO 2020/022416
Patent Literature 3: WO 2022/181670

### SUMMARY OF THE INVENTION

### PROBLEMS TO BE SOLVED BY THE INVENTION

A main object of the present invention is to provide a method capable of selectively obtaining a desired compound with high yield while shortening the reaction time in the regeneration of a nitrile compound through dehydration of an amide compound, and also of suppressing the generation of by-products.

Another main object of the present invention is to realize an efficient method for producing a carbonate ester by applying such a method for producing a nitrile compound.

### MEANS FOR SOLVING THE PROBLEMS

The present inventors have studied a method for producing a furonitrile compound by dehydrating a furamide compound, and have found that, when the furamide compound and a diluting agent are brought into contact with a catalyst in a gas phase, the reaction rate can be significantly improved to shorten the reaction time, while suppressing the generation of by-products and selectively obtaining a desired compound with high yield.

According to the present invention, the regeneration rate of a furonitrile compound from a furamide compound through a dehydration reaction can be improved. Also, in a method for producing a carbonate ester of the present invention using carbon dioxide and an alcohol, which incorporates such a dehydration reaction step, excellent effects can be achieved, such as enabling efficient production of the carbonate ester.

For example, the present invention includes the following.

[1] A method for producing a furonitrile compound, the method comprising a dehydration reaction represented by the following formula (1), in which a furamide compound is dehydrated,
wherein the dehydration reaction has a contact step in which the furamide compound and a diluting agent are brought into contact with a catalyst in a gas phase:

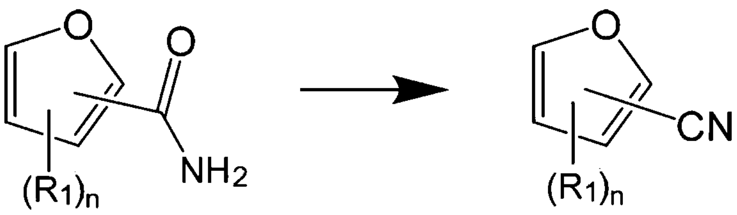

(1)

in formula (1), $R_1$ represents a hydrogen atom, a halogen atom, an alkyl group having 1 to 20 carbon atoms and optionally having a substituent, an alkoxyl group having 1 to 20 carbon atoms and optionally having a substituent, a cycloalkyl group having 6 to 20 carbon atoms and optionally having a substituent, an aryl group having 6 to 20 carbon atoms and optionally having a substituent, a cycloalkoxyl group having 6 to 20 carbon atoms and optionally having a substituent, an aryloxy group having 6 to 20 carbon atoms and optionally having a substituent, or a cyclic compound group of a 3- to 20-membered ring containing at least two types of different elements and optionally having a substituent; and
n represents an integer of 0 to 3.

[2] The method for producing a furonitrile compound according to the above [1], wherein $R_1$ is a hydrogen atom, an alkyl group having 1 to 20 carbon atoms, a cycloalkyl group having 6 to 20 carbon atoms, or an aryl group having 6 to 20 carbon atoms.
[3] The method for producing a furonitrile compound according to the above [1] or [2], wherein a temperature at which the furamide compound and the diluting agent are brought into contact with the catalyst in the contact step is 150°C or higher.
[4] The method for producing a furonitrile compound according to any of the above [1] to [3], such as the above [1], wherein a pressure in a reaction system in the contact step is 101.3 kPa or lower.
[5] The method for producing a furonitrile compound according to any of the above [1] to [4], such as the above [1], wherein a contact time of the furamide compound with the catalyst in the gas phase is 0.001 seconds or longer and shorter than 10 seconds.
[6] The method for producing a furonitrile compound according to any of the above [1] to [5], such as the above [1], wherein the catalyst comprises an alkali metal.
[7] The method for producing a furonitrile compound according to any of the above [1] to [6], such as the above [1], wherein the diluting agent comprises at least any of a solvent and an inert gas.
[8] The method for producing a furonitrile compound according to the above [7], wherein the solvent comprises at least an aromatic compound.
[9] The method for producing a furonitrile compound according to the above [8], wherein the aromatic compound is represented by the following formula (2):

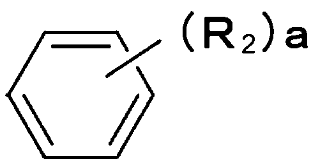

(2)

wherein

$R_2$ is each independently selected from an alkyl group having 1 to 10 carbon atoms and optionally having a substituent, an alkoxy group having 1 to 10 carbon atoms and optionally having a substituent, an aryl group having 6 to 30 carbon atoms and optionally having a substituent, and an aryl group having 6 to 30 carbon atoms and

optionally having a substituent; and
a is an integer of 1 to 6.

[10] The method for producing a furonitrile compound according to the above [8] or [9], such as the above [8], wherein the aromatic compound has one or two aromatic rings and one or more selected from an alkyl group, an alkoxy group, and an aryloxy group.
[11] The method for producing a furonitrile compound according to any of the above [8] to [10], such as the above [8], wherein the aromatic compound comprises at least any of phenyl ether, 1,3,5-trimethylbenzene, p-cymene, and anisole.
[12] The method for producing a furonitrile compound according to any of the above [7] to [11], such as the above [7], wherein the solvent is compatible with the furamide compound.
[13] The method for producing a furonitrile compound according to any of the above [7] to [12], such as the above [7], wherein an inert gas in a vaporized state is used in the contact step.
[14] The method for producing a furonitrile compound according to any of the above [7] to [13], such as the above [7], wherein the inert gas comprises at least nitrogen gas.
[15] A method for producing a carbonate ester, the method comprising:

a first reaction step that comprises a carbonate ester production reaction in which an alcohol is allowed to react with carbon dioxide in the presence of an furonitrile compound to produce a carbonate ester and water, and a hydration reaction in which the furonitrile compound is hydrated with the produced water to produce a furamide compound; and
a second reaction step in which the furamide compound is separated from a reaction system of the first reaction step and then the furamide compound is regenerated into a furonitrile compound by means of a dehydration reaction in which the furamide compound is dehydrated, the dehydration reaction having the contact step according to any of the above [1] to [14],
wherein at least a part of the furonitrile compound regenerated in the second reaction step is used in the first reaction step.

[16] The method for producing a carbonate ester according to the above [15], wherein a catalyst comprising cerium oxide is used in the carbonate ester production reaction.
[17] The method for producing a carbonate ester according to the above [15] or [16], such as the above [15], wherein the alcohol comprises an alcohol having 1 to 6 carbon atoms.

## ADVANTAGEOUS EFFECT OF THE INVENTION

According to the present invention, the production (regeneration) of an aromatic nitrile compound, such as furonitrile or an analogue thereof, from an aromatic amide compound, such as furamide or an analogue thereof, can be efficiently carried out. That is, in the above-described dehydration reaction of a furamide compound for regeneration, the generation of by-products can be suppressed, a desired compound can be selectively obtained with high yield, and a reaction rate can be improved.
For this reason, according to the present invention, the reaction time of the dehydration reaction of a furamide compound for regeneration can be significantly shortened when compared to conventional methods. Furthermore, in the dehydration step using a gas phase reaction, the size of the reaction container can be reduced as compared with conventional dehydration steps for aromatic amide compounds such as those involving liquid phase reactions. Therefore, according to the present invention, it becomes possible to easily industrialize the step of regenerating an aromatic nitrile compound, such as a furonitrile compound, from an aromatic amide compound, such as a furamide compound.
In addition, according to the present invention, by producing a furonitrile compound as described above, an efficient method for producing a carbonate ester can also be realized.

## BRIEF DESCRIPTION OF DRAWINGS

[Figure 1] Figure 1 shows an example of an apparatus for producing a carbonate ester.
[Figure 2] Figure 2 is a schematic diagram showing an apparatus for producing 2-furonitrile, having a vaporizer and a reactor, in Examples.

DESCRIPTION OF EMBODIMENTS

Hereinafter, suitable embodiments of the present invention will be described in detail.

<1. Method for Producing Furonitrile Compound>

In the method for producing a furonitrile compound of the present invention, an aromatic amide compound, such as a furamide compound, is dehydrated in a gas phase, thereby converting it into an aromatic nitrile compound, namely a furonitrile compound. That is, in the method for producing a furonitrile compound of the present invention, for example, a dehydration reaction is caused by a contact step in which a furamide compound is brought into contact with a catalyst carrying a basic metal oxide in a gas phase, preferably at a high temperature, thereby producing a furonitrile compound.

(Substrate for Dehydration Reaction)

The dehydration reaction of a furamide compound in the method for producing a furonitrile compound is represented by the following formula (1).

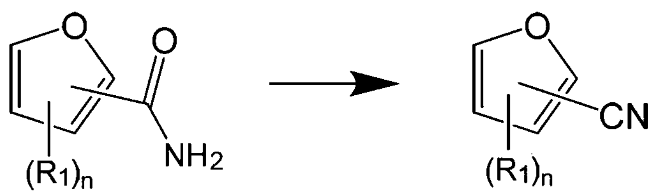

(1)

In the above formula (1), $R_1$ represents a hydrogen atom, a halogen atom, an alkyl group having 1 to 20 carbon atoms and optionally having a substituent, an alkoxyl group having 1 to 20 carbon atoms and optionally having a substituent, a cycloalkyl group having 6 to 20 carbon atoms and optionally having a substituent, an aryl group having 6 to 20 carbon atoms and optionally having a substituent, a cycloalkoxyl group having 6 to 20 carbon atoms and optionally having a substituent, an aryloxy group having 6 to 20 carbon atoms and optionally having a substituent, or a cyclic compound group of a 3- to 20-membered ring containing at least two types of different elements and optionally having a substituent.

Examples of the above-described substituent that may be included in $R_1$ include a halogen atom, a hydroxy group, a carboxy group, a cyano group, an amide group having 1 to 10 carbon atoms, an alkyl group having 1 to 10 carbon atoms, a cycloalkyl group having 5 to 10 carbon atoms, and an alkoxy group having 1 to 10 carbon atoms. Note that the number of carbon atoms in $R_1$ of the general formula (1) described above refers to the total number of carbon atoms, including those in the substituent.

Also, n in the above formula (1) represents an integer of 0 to 3.

In the case where n in the formula (1) is 2 or more and the furamide compound has a plurality of substituents $R_1$, the plurality of $R_1$ are each independently selected from the above-described options. The same applies to the plurality of substituents $R_1$ in the furonitrile compound.

$R_1$ in the formula (1) is preferably a hydrogen atom, an alkyl group having 1 to 20 carbon atoms, a cycloalkyl group having 6 to 20 carbon atoms, or an aryl group having 6 to 20 carbon atoms.

$R_1$ in the formula (1) is more preferably a hydrogen atom, an alkyl group having 1 to 5 carbon atoms, a cycloalkyl group having 6 to 12 carbon atoms, or an aryl group having 6 to 12 carbon atoms.

$R_1$ in the formula (1) is still more preferably any of a hydrogen atom, a methyl group, an ethyl group, an isopropyl group, a n-propyl group, a t-butyl group, a n-butyl group, an isobutyl group, a cyclohexyl group, a phenyl group, and a naphthyl group.

$R_1$ in the formula (1) is particularly preferably any of a hydrogen atom, a methyl group, and an ethyl group.

Also, n in the above formula (1) is preferably any of 0 to 2, more preferably 0 or 1, and still more preferably 0.

Preferred specific examples of the furamide compound include furamide, such as 2-furamide and 3-furamide, and preferred specific examples of the furonitrile compound include furonitrile, such as 2-furonitrile and 3-furonitrile.

(Other Reaction Substrates)

As described above, the furonitrile compound is obtained by a dehydration reaction of the furamide compound. However, in methods for producing aromatic nitrile compounds other than the above-described furonitrile compound, examples of aromatic amide compounds that can be used include amide compounds having an aromatic hydrocarbon ring such as a benzene ring, a naphthalene ring, or an anthracene ring, or a heteroaryl ring. Among these aromatic amide compounds, an aromatic amide compound having a heteroaryl ring, i.e., a heteroaryl amide compound is preferably used,

and examples of the heteroaryl amide compound include amide compounds having a pyridine ring, a pyrimidine ring, a pyridazine ring, a pyrazine ring, a triazine ring, a pyrrole ring, a furan ring, a thiophene ring, an imidazole ring, a pyrazole ring, an oxazole ring, or the like.

Preferred specific examples of the heteroaryl amide compound include those having a pyridine ring such as pyridinecarboamide (2-pyridinecarboamide, 3-pyridinecarboamide, and 4-pyridinecarboamide) described above.

Also, the compounds to be produced by the production method of the present invention may include aromatic nitrile compounds other than the furonitrile compound. Examples of the aromatic nitrile compounds other than the furonitrile compound include products of a dehydration reaction corresponding to the above-described aromatic amide compounds. Accordingly, specific examples of the aromatic nitrile compounds targeted in the production method of the present invention include heteroaryl nitrile compounds. Preferred specific examples of the heteroaryl nitrile compounds include those having a pyridine ring such as cyanopyridine (2-cyanopyridine, 3-cyanopyridine, and 4-cyanopyridine).

(Catalyst)

In this regard, the catalyst to be used in the above-described dehydration reaction in the present invention preferably comprises an oxide of an alkali metal (K, Li, Na, Rb, or Cs). In particular, as the catalyst to be used in the above-described reaction, it is preferable to use a catalyst comprising an oxide of at least any of Na, K, Rb, and Cs (cesium). Also, as a carrier of the above-described catalyst, a substance generally serving as a catalyst carrier can be used, but as a result of examination of various carriers, it is preferable that the carrier comprises any of $SiO_2$ and $ZrO_2$.

An example of the method for producing the catalyst to be used in the above-described dehydration reaction in the present invention will be described below. In the case where the carrier is $SiO_2$, commercially available powdered or spherical $SiO_2$ can be used, and it is preferable that the particle size is adjusted to, for example, 4.0 mm or less so that an active metal can be uniformly carried, and that pre-baking is carried out in the air at 700°C for 1 hour for removing moisture. In addition, although there are $SiO_2$ products with various characteristics, the larger the specific surface area is, the better it is because the active metal can be highly dispersed and the amount of the furonitrile compound produced is improved. Specifically, the specific surface area is preferably 300 $m^2/g$ or more. However, the specific surface area of the catalyst after preparation may be reduced to be less than the specific surface area of $SiO_2$ alone due to the interaction between $SiO_2$ and the active metal or the like. In such a case, the specific surface area of the catalyst after the production is preferably 150 $m_2/g$ or more. A metal oxide serving as an active species can be carried according to an impregnation method such as an incipient wetness method and an evaporation drying method.

It is preferable that a metal salt that serves as a precursor of the catalyst is watersoluble, and when it is an alkali metal, various compounds such as a carbonate, a hydrogencarbonate, a chloride salt, a nitrate, and a silicate can be used. A carrier is impregnated with an aqueous solution of a precursor of a basic metal, followed by drying and baking, and then the obtained product can be used as the catalyst. The baking temperature varies depending on the precursor to be used, but it is preferably 400 to 600°C.

Furthermore, as the catalyst to be used in the present invention, a catalyst in which only one or more alkali metal oxides are carried on a carrier composed of one or both of $SiO_2$ and $ZrO_2$ is preferable, but other than the above-described elements, the catalyst may contain an unavoidable impurity mixed during the catalyst production process or the like. However, it is desirable that mixing of an impurity is suppressed as much as possible.

In this regard, the catalyst to be suitably used in the present invention, wherein a metal oxide serving as an active species is carried on a carrier, may be in the form of either powder or a molded body, and in the case of the molded body, it may be any of a spherical type, a pellet type, a cylinder type, a ring type, a wheel type, a granular type, and the like. Also, the catalyst can be fixed to a support structure such as a honeycomb and used.

The size of the carrier is not restricted, but in the case where a spherical carrier is used, the average diameter of particles of the catalyst (carrier diameter) is preferably in the range of 0.01 to 8.0 mm, more preferably 0.03 to 6.0 mm, still more preferably 0.05 to 5.0 mm, particularly preferably 0.07 to 1.0 mm, and even more preferably 0.075 to 0.150 mm. By using the catalyst whose carrier diameter is relatively larger than the average diameter (carrier diameter) of particles of a catalyst to be used in a liquid phase reaction as described above, the flow channel of the reaction substrate in the gas phase reaction is ensured, and a high space velocity can be provided. Note that in the case where a fluidized bed is used in the gas phase reaction, a catalyst with a relatively small particle diameter is preferably used so that the catalyst can be stirred with a small gas flow amount, and in the case where a fixed bed is used in the gas phase reaction, a relatively large particle diameter or shape is preferable so that the generation of pressure loss can be prevented as much as possible.

The carrier diameter, i.e., the average diameter of particles of the catalyst refers to the average diameter of particles of the whole catalyst including a catalytic component of the catalyst and the carrier. The value of the carrier diameter of the catalyst is measured based on the sieving method: Test sieving - General requirements defined in JIS Z 8815 or the like.

Also, the carrying amount of the catalyst may be set as appropriate, but based on the total catalyst weight, the carrying amount in metal conversion of the active species such as an alkali metal oxide is preferably 0.05 to 2.0 mmol/g,

more preferably 0.10 to 1.5 mmol/g, and still more preferably 0.30 to 1.0 mmol/g. In addition, the amount of the catalyst used during the reaction may also be set as appropriate.

(Reaction System and Reaction Container)

In the method for producing a furonitrile compound of the present invention, it is preferable to use a gas phase reaction in which an amide compound in the form of gas or mist is allowed to flow through a catalyst layer together with a diluting agent, and for example, a gas phase reaction apparatus with a fixed bed or fluidized bed can be applied thereto. Thus, in the contact step in which the furamide compound is brought into contact with the catalyst in the gas phase, the furamide compound in a completely vaporized state is preferably used, but the furamide compound in a state where mist is partially included in gas may also be used.

It is desirable that the method for producing a furonitrile compound is carried out while removing produced by-product water by means of a dehydration reaction. The present inventors diligently made researches and found that, for example, when a vaporizing chamber is attached to the upper portion of a reaction tube in which the catalyst is put, the furamide compound is dropped into the vaporizing chamber and the amide compound is allowed to pass through the catalyst layer in the gas phase in a short time, the amount of the furonitrile compound produced can be improved, and the production of by-products can be suppressed.

(Diluting Agent)

In the above-described dehydration reaction, it is preferable to use a diluting agent, and in particular, it is preferable to use at least any of an inert gas and a solvent, which will be described in detail later, as the diluting agent in the dehydration reaction. The diluting agent is mainly used to reduce the frequency of contact between water produced when the furamide compound is vaporized and allowed to react with the catalyst, and the unreacted furamide compound or the reaction product, i.e., the furonitrile compound, thereby suppressing the production of by-products such as pyridine.

Therefore, an inert gas can be used as the diluting agent. Also, when a diluting agent that is compatible with the furamide compound in the liquid phase is used, in the step of vaporizing the furamide compound, by mixing the furamide compound with a solvent compatible therewith in advance and delivering the mixture, the trouble of closure due to precipitation of the furamide compound can be prevented. That is, as the diluting agent, an inert gas may be used or a liquid that is incompatible with the furamide compound as the target of the dehydration reaction may be used, and it is more preferable to use a liquid that is compatible therewith. The diluting agent compatible with the furamide compound in the liquid phase includes not only a diluting agent that can be mixed with the furamide compound in any ratio, but also a diluting agent that can dissolve the furamide compound only to a predetermined degree of solubility, in an amount by which the furamide compound can be dissolved to a concentration that is equal to or lower than the upper limit value of the degree of solubility at a predetermined temperature.

(Inert Gas)

As described above, in the dehydration reaction, it is preferable that an inert gas is brought into contact with the catalyst together with the furamide compound in the gas phase. That is, the inert gas is preferably used in a vaporized state in the dehydration reaction. Specific examples of the inert gas to be used in the dehydration reaction include nitrogen gas and rare gases such as helium and argon, and nitrogen gas is preferably used. The flow rate, etc. of the inert gas will be described later.

(Solvent)

In the above-described dehydration reaction, it is preferable to use a solvent. In particular, as described above, in the step of vaporizing the furamide compound, by mixing the furamide compound with a solvent compatible therewith in advance and delivering the mixture, the trouble of closure due to precipitation of the furamide compound can be prevented. That is, it is preferable to use a solvent that is compatible with the furamide compound as the target of the dehydration reaction. The solvent compatible with the furamide compound includes not only a solvent that can be mixed with the furamide compound in any ratio, but also a solvent that can dissolve the furamide compound as the target only to a predetermined degree of solubility, in an amount by which the furamide compound can be dissolved to a concentration that is equal to or lower than the upper limit value of the degree of solubility at a predetermined temperature.

The solvent preferably comprises at least an aromatic compound, and as the aromatic compound, a compound represented by the following formula (2) is suitably used.

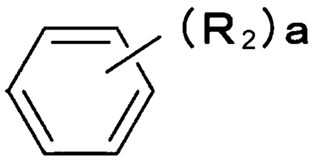

(2)

In the formula (2), $R_2$ is each independently selected from an alkyl group having 1 to 10 carbon atoms and optionally having a substituent, an alkoxy group having 1 to 10 carbon atoms and optionally having a substituent, an aryl group having 6 to 30 carbon atoms and optionally having a substituent, and an aryl group having 6 to 30 carbon atoms and optionally having a substituent.

$R_2$ in the formula (2) is preferably an alkyl group having 1 to 6 carbon atoms and optionally having a substituent, an alkoxy group having 1 to 6 carbon atoms and optionally having a substituent, or an aryl group having 6 to 18 carbon atoms and optionally having a substituent. $R_2$ is more preferably any of an alkyl group having 1 to 4 carbon atoms and optionally having a substituent, an alkoxy group having 1 to 3 carbon atoms and optionally having a substituent, and an aryl group having 6 to 12 carbon atoms and optionally having a substituent, and still more preferably any of an alkyl group having 1 to 3 carbon atoms, an alkoxy group having 1 or 2 carbon atoms and optionally having a substituent, and an aryl group having 6 to 8 carbon atoms and optionally having a substituent.

In the formula (2), a is an integer of 1 to 6, and a is preferably an integer of 1 to 4, and more preferably an integer of 1 to 3.

Examples of the above-described substituent include a halogen atom, a hydroxy group, a carboxy group, a cyano group, an amide group having 1 to 10 carbon atoms, an alkyl group having 1 to 10 carbon atoms, a cycloalkyl group having 5 to 10 carbon atoms, and an alkoxy group having 1 to 10 carbon atoms. Note that the number of carbon atoms in the general formula (2) refers to the total number of carbon atoms, including those in the substituent.

The aromatic compound as the solvent preferably has one or two aromatic rings and one or more selected from an alkyl group, an alkoxy group, and an aryloxy group, and more preferably has 1 to 3 alkyl groups and/or alkoxy groups in total. Also, the aromatic compound preferably has a plurality of, such as 2, aromatic rings, for example benzene rings, connected via an ether bond.

The aromatic compound serving as the solvent to be used in the dehydration reaction preferably comprises at least any of phenyl ether, 1,3,5-trimethylbenzene, p-cymene, and anisole. In addition, other specific examples of the solvent include aromatic compounds such as pyridine compounds, ketone compounds, ether compounds, ester compounds, and alcohols, among which the above-described aromatic compounds are preferably used.

Examples of the furan compounds serving as the solvent, that is, compounds having a furan skeleton, include dimethoxyfuran.

Also, examples of the ketone compounds serving as the solvent include cyclic ketone compounds such as cyclopentanone and cyclohexane, and acetone.

Note that, as the solvent for the dehydration reaction, it is preferable to use a solvent composed of only one or more substances selected from the above-described compounds, but a mixed solvent further containing another compound may also be used.

The solvent is preferably vaporized together with the furamide compound and used in the dehydration reaction (contact step). In this way, the use of a solvent that is vaporized and used in the dehydration reaction is preferable. Also, as will be described in detail later, since the solvent is used in the dehydration reaction suitably carried out at a high temperature, the solvent, preferably an aromatic compound, preferably has a high autoignition temperature. For example, the solvent preferably has an autoignition temperature of 300°C or higher, 340°C or higher, 380°C or higher, or 400°C or higher, more preferably 430°C or higher or 450°C or higher, still more preferably 470°C or higher or 500°C or higher, and particularly preferably 550°C or higher.

In addition, although not particularly limited, the boiling point of the solvent, preferably an aromatic compound, is, for example, 20°C or higher and 300°C or lower, preferably 80°C or higher and 250°C or lower, and more preferably 110°C or higher and 200°C or lower at ordinary pressure.

(Conditions for Dehydration Reaction Including Contact step)

As described above, the method for producing a furonitrile compound has a contact step in which the furamide compound is brought into contact with the catalyst in the gas phase during the dehydration reaction.

In the contact step, the temperature at which the furamide compound and the diluting agent such as solvent are brought into contact with the catalyst is, for example, 150°C or higher, preferably 180°C or higher, more preferably 200°C or

higher, still more preferably 220°C or higher, and particularly preferably 250°C or higher or 270°C or higher. The contact temperature is, for example, 150°C or higher and 550°C or lower, more preferably 200°C or higher and 520°C or lower, still more preferably 220°C or higher and 500°C or lower, and particularly preferably 250°C or higher and 490°C or lower or 270°C or higher and 480°C or lower.

Note that the temperature at which the furamide compound or the like is brought into contact with the catalyst refers to, for example, the temperature in the vicinity of the catalyst layer in a reaction tube (reaction container) to which the catalyst is fixed.

The contact time of the furamide compound with the catalyst in the contact step, and in the case where an inert gas, a solvent, and the like are included together with the furamide compound, the contact time of a mixed gas of gaseous composition thereof with the catalyst is preferably 0.001 seconds or longer or 0.005 seconds or longer, for example, 0.01 seconds or longer and shorter than 10 seconds. The above-described contact time with the catalyst is more preferably 0.1 seconds or longer and shorter than 5 seconds, and still more preferably 0.5 seconds or longer and shorter than 2 seconds.

Note that the contact time with the catalyst is the average time during which the gas of the furamide compound or the above-described mixed gas passes through the catalyst layer, and it is calculated from: contact time with catalyst (sec) = height of catalyst layer in reactor (m) ÷ linear velocity of gas in reactor (m/sec).

In the contact step, the molar ratio between the flow rate of the furamide compound and the flow rate of the gaseous composition of the diluting agent such as inert gas or solvent is preferably 1:0 to 1:200, more preferably 1:1 to 1:100, and still more preferably 1:1 to 1:20.

The space velocity (SV) of the whole gas component in the contact step is preferably 1,000 to 50,000 ($h^{-1}$), more preferably 1,500 to 30,000 ($h^{-1}$), and still more preferably 2,000 to 25,000 ($h^{-1}$). Also, when carrying out the contact step under reduced pressure, the space velocity (SV) of the whole gas component is preferably 1,000 to 1,000,000 ($h^{-1}$), more preferably 1,500 to 700,000 ($h^{-1}$), and still more preferably 1,900 to 504,000 ($h^{-1}$).

As the conditions for the dehydration reaction, the pressure may be increased pressure (for example, 506.5 (kPa)) to ordinary pressure (101.3 (kPa)), or reduced pressure (for example, 101.3 to 1.0 (kPa) or lower, for example, reduced pressure in stages such as 101.3 to 1.0 (kPa) to 0.1 (kPa)), and is not particularly restricted thereto. However, it is preferable to carry out the dehydration reaction under reduced pressure.

The reaction pressure in the dehydration reaction is preferably 101.3 (kPa) or lower, more preferably 90 (kPa) to 1.0 (kPa) or 80 (kPa) to 5.0 (kPa), still more preferably 70 (kPa) to 10 (kPa) or 60 (kPa) to 20 (kPa), and particularly preferably 50 (kPa) to 30 (kPa).

It is also preferable that the furamide compound is dehydrated when it is in the form of liquid before vaporized. In the case where a molecular sieve is used as a dehydrating agent, the type and form thereof are not particularly restricted, but for example, spherical or pellet-type molecular sieves 3A, 4A, 5A, etc. generally having high water absorbability can be used. For example, ZEOLUM manufactured by Tosoh Corporation can be suitably used.

(Example of by-Product in Dehydration Reaction)

It is considered that in the dehydration reaction of the furamide compound, furan, etc. are by-produced due to decomposition of the furamide compound. However, almost no by-product such as furan described above is contained in the reaction solution after the dehydration reaction using the contact step of the present invention.

<2. Method for Producing Carbonate Ester Using Furonitrile Compound>

By providing the contact step in the gas phase to the regeneration of the furamide compound into the furonitrile compound by means of the dehydration reaction, the reaction rate was successfully improved significantly to reduce the reaction time significantly. By this, it became possible to achieve a balance between the rate of regeneration of the furamide compound into the furonitrile compound by means of the dehydration reaction and the rate of synthesis of a carbonate ester from $CO_2$ and an alcohol using the furonitrile compound, and it became possible to establish these reactions as a series of commercial processes. Accordingly, by applying this finding to a method for producing a carbonate ester, the present inventors successfully conceived the below-described method for producing a carbonate ester.

(First Reaction Step)

The first reaction step in the method for producing a carbonate ester of the present invention includes a reaction in which an alcohol is allowed to directly react with carbon dioxide in the presence of a solid catalyst containing, for example, $CeO_2$ (cerium oxide), etc. and the furonitrile compound to produce the carbonate ester (carbonate ester production reaction).

In this step, when the alcohol is allowed to react with carbon dioxide, water is also produced in addition to the carbonate ester, and by a hydration reaction of the furonitrile compound present in the reaction system and produced

water, a furamide compound is produced, and the produced water can be removed from the reaction system or reduced. By efficiently removing water from the reaction system in this way, the production of the carbonate ester can be promoted. For example, it is as shown in the following formula.

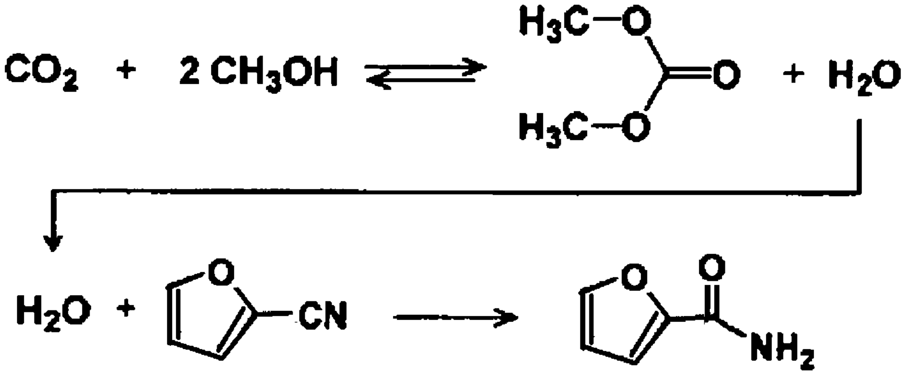

(Alcohol)

In this regard, as the alcohol, any alcohol selected from one or more of a primary alcohol, a secondary alcohol, and a tertiary alcohol can be used. For example, methanol, ethanol, 1-propanol, isopropanol, 1-butanol, 1-pentanol, 1-hexanol, 1-heptanol, 1-octanol, 1-nonanol, allyl alcohol, 2-methyl-1-propanol, cyclohexanemethanol, benzyl alcohol, ethylene glycol, 1,2-propanediol and 1,3-propanediol are preferably used because a high yield of a product and a high reaction rate are obtained. In these cases, carbonate esters produced are respectively dimethyl carbonate, diethyl carbonate, dipropyl carbonate, diisopropyl carbonate, dibutyl carbonate, dipentyl carbonate, dihexyl carbonate, diheptyl carbonate, dioctyl carbonate, dinonane carbonate, diallyl carbonate, di-2-methyl-propyl carbonate, dicyclohexanemethyl carbonate, dibenzyl carbonate, ethylene carbonate, 1,2-propylene carbonate and 1,3-propylene carbonate.

Also, in the first reaction step, an alcohol having 1 to 6 carbon atoms is preferably used, and an alcohol having 2 to 4 carbon atoms is more preferably used. In particular, in the case where the carbonate ester obtained is used as a raw material of diaryl carbonate, the number of carbon atoms in the alcohol is preferably adjusted within the above-described range.

In addition, it is preferable to use a monohydric alcohol or a dihydric alcohol in the first reaction step.

(Catalyst for Production of Carbonate Ester)

Also, in the first reaction step in the production of the carbonate ester, it is preferable to use one or both of $CeO_2$ and $ZrO_2$ as a solid catalyst. For example, it is preferable to use only $CeO_2$, only $ZrO_2$, a mixture of $CeO_2$ and $ZrO_2$, a solid solution or composite oxide of $CeO_2$ and $ZrO_2$, or the like, and it is particularly preferable to use only $CeO_2$. Also, the mixing ratio between $CeO_2$ and $ZrO_2$ in the solid solution or composite oxide thereof is basically 50:50, but can be changed as appropriate.

In this regard, the catalyst to be used in the first reaction step may be in the form of either powder or a molded body, and in the case of the molded body, it may be any of a spherical type, a pellet type, a cylinder type, a ring type, a wheel type, a granular type, and the like.

(Carbon Dioxide)

As carbon dioxide to be used in the present invention, not only carbon dioxide prepared as industrial gas, but also carbon dioxide separated and recovered from exhaust gas of plants producing various products, ironworks, power plants, etc. can be used.

(Solvent for Carbonate Ester Production Reaction)

In the carbonate ester production reaction, it is preferable to use a solvent having a boiling point higher than that of the amide compound to be produced. More preferably, the solvent in the carbonate ester production reaction comprises at least one of dialkylbenzene, alkylnaphthalene, and diphenylbenzene. Specific examples thereof include Barrel Process Oil B28AN and Barrel Process Oil B30 (manufactured by Matsumura Oil Co., Ltd.), each of which contains components including dialkylbenzene, alkylnaphthalene, diphenylbenzene, etc.

(Separation by Distillation)

After the reaction, a carbonate ester as the main product, a furamide compound as a by-product, an unreacted furonitrile compound, and a solid catalyst such as $CeO_2$ are separated by distillation, thereby recovering the products.

(Second Reaction Step)

Next, the second reaction step in the present invention will be described. In the second reaction step, the furamide compound by-produced in the first reaction step is preferably separated from the system obtained after the carbonate ester production reaction, and then a furonitrile compound is produced by means of a dehydration reaction. The second reaction step corresponds to the above-described method for producing a furonitrile compound. That is, in the second reaction step for the production of the carbonate ester, the furonitrile compound is produced from the furamide compound according to the technique described in the column regarding the above-described method for producing a furonitrile compound, thereby regenerating the furonitrile compound. Therefore, the details of the second reaction step are omitted.

(Reuse of Furonitrile Compound)

The furonitrile compound regenerated in the second reaction step can be reused in the first reaction step (hydration reaction).

According to the present invention, as described above, by carrying out the dehydration reaction of the furamide compound in the gas phase, the furonitrile compound can be efficiently regenerated from the furamide compound while suppressing the generation of by-products. Furthermore, by fixing the catalyst in a reaction tube, for example, the step for subjecting the catalyst to solid-liquid separation is made unnecessary and the furonitrile compound can be easily recovered. Thus, in the present invention, it is possible to selectively regenerate the furonitrile compound from the furamide compound, and to promote a series of reactions while separating the respective components only by distillation without solid-liquid separation of the catalyst. Accordingly, an efficient process, which will be described in detail later, can be realized.

<3. Apparatus for Producing Carbonate Ester>

Hereinafter, an apparatus for producing a carbonate ester to be used in the present invention will be described in detail by way of a specific example. Figure 1 shows an example of a suitable facility. In the following specific example, 2-furamide is used as the furamide compound, and 2-furonitrile is used as the furonitrile compound; however, the present invention is not limited thereto.

(First Reaction Step)

In the first reaction step, raw materials, i.e., an alcohol (1-propanol (PrOH); liquid phase), 2-furonitrile (2-FN; liquid phase), and carbon dioxide ($CO_2$; liquid phase, it may be supplied via a booster pump) are continuously supplied to a buffer tank 1 using a raw material feed piping 31, a $CO_2$ recovery column top $CO_2$ transfer piping ($CO_2$ transfer piping at the top side of the $CO_2$ recovery column) 15, a carbonate ester recovery column top liquid transfer piping (liquid transfer piping at the top side of the carbonate ester recovery column) 19, and an amide separation column top liquid transfer piping (liquid transfer piping at the top side of the amide separation column) 21. This mixed solution of the raw materials is circulated between a fixed bed flow-type carbonate ester reactor 2 in which a solid catalyst (solid phase) composed of, for example one or both of $CeO_2$ and $ZrO_2$ is fixed to a support material (first reaction portion) and the buffer tank 1 via a first reaction solution circulation piping 11 and a second reaction solution circulation piping 12 using a pump (not shown), thereby synthesizing dipropyl carbonate (DPrC). The reaction solution containing DPrC is continuously withdrawn from the second reaction solution circulation piping 12 in the same amount as the amount of the raw materials supplied to the buffer tank 1, recovered using a piping 13, and delivered to a DPrC recovery step. PrOH and $CO_2$ are recovered from the reaction solution and transferred to the buffer tank 1 respectively via the carbonate ester recovery column top liquid transfer piping 19 and the $CO_2$ recovery column top $CO_2$ transfer piping 15, thereby reusing them. At the start of the reaction, for example, new 2-furonitrile is used, but 2-furonitrile regenerated from 2-furamide may be separated and purified in an amide separation column 6 and transferred to the buffer tank 1 via the amide separation column top liquid transfer piping 21, thereby reusing it.

As a direct synthesis apparatus for a carbonate ester using for example, $CeO_2$, $ZrO_2$, etc. as a solid catalyst (carbonate ester reactor 2), any of flow reactors such as a batch reactor, a semi-batch reactor, a continuous tank reactor, and a tube reactor may be used. When a catalyst is fixed to a reactor, it is not necessary to filter and separate the catalyst.

For this reason, a fixed bed reactor is preferable.

(Reaction Solution Temperature)

The reaction solution temperature in the carbonate ester reactor 2 is preferably 50 to 300°C. When the reaction solution temperature is lower than 50°C, the reaction rate is low, the carbonate ester synthesis reaction and the hydration reaction with 2-furonitrile hardly progress, and the productivity of the carbonate ester tends to be low. When the reaction solution temperature is higher than 300°C, the reaction rate of each reaction is high, but the carbonate ester is easily decomposed or denatured and 2-furamide easily reacts with an alcohol. For this reason, the yield of the carbonate ester tends to be low. The reaction solution temperature is more preferably 100 to 150°C. However, since it is considered that the ideal reaction solution temperature varies depending on the type and amount of the solid catalyst and the amount and ratio of the raw materials (alcohol and 2-furonitrile), it is desirable to set optimum conditions as appropriate. Since the preferred reaction solution temperature is 100 to 150°C, it is also desirable to preheat the raw materials (such as alcohol and 2-furonitrile) with steam or the like on a stage before the carbonate ester reactor.

(Reaction Pressure)

The reaction pressure in the carbonate ester reactor 2 is preferably 0.1 to 20 MPa (absolute pressure). When the reaction pressure is lower than 0.1 MPa (absolute pressure), a decompression device is required. As a result, facilities are complicated and the cost is increased, and in addition, a power energy for reducing the pressure is required, resulting in decrease of the energy efficiency. Also, when the reaction pressure is higher than 20 MPa, the hydration reaction with 2-furonitrile does not easily progress, resulting in decrease of the yield of the carbonate ester. In addition, a power energy for increasing the pressure is required, resulting in decrease of the energy efficiency. From the viewpoint of increasing the yield of the carbonate ester, the reaction pressure is more preferably 0.5 to 15 MPa (absolute pressure), and still more preferably 1.0 to 10 MPa (absolute pressure).

(Amount of 2-Furonitrile)

The aromatic nitrile compound to be used for the hydration reaction, such as 2-furonitrile, is preferably used in a molar quantity that is 0.1 to 5 times the theoretical molar quantity of water by-produced by the reaction of the alcohol and $CO_2$ as the raw materials, and it is desirably introduced into the reactor before the reaction. The molar quantity of the aromatic nitrile compound, such as 2-furonitrile, is more desirably 0.2 to 3 times, and particularly desirably 0.3 to 1.5 times the theoretical molar quantity of water by-produced by the reaction of the alcohol and $CO_2$ as the raw materials. In the case where the molar quantity of the aromatic nitrile compound, such as 2-furonitrile, is too small, since the amount of 2-furonitrile or the like contributing to the hydration reaction is small, the yield of the carbonate ester may be decreased. Meanwhile, in the case where the aromatic nitrile compound, such as 2-furonitrile, is introduced in an excess molar quantity relative to the alcohol as the raw material, the side reaction of 2-furonitrile or the like is increased, and therefore it is undesirable. Furthermore, since it is considered that the ideal amounts of the alcohol and 2-furonitrile or the like relative to the solid catalyst vary depending on the type and amount of the solid catalyst, the type of the alcohol, and the ratio between the alcohol and 2-furonitrile, it is desirable to set optimum conditions as appropriate.

(Separation of Reaction Products)

Preferably, the separation of reaction products is entirely carried out by means of distillation. After the reaction in the carbonate ester reactor 2, a reaction solution 13 is transferred to a $CO_2$ recovery column 3. A mixture of PrOH, DPrC, 2-furonitrile, and 2-furamide is recovered from the bottom of the $CO_2$ recovery column 3 via a $CO_2$ recovery column bottom liquid transfer piping 14, and $CO_2$ is recovered from the top of the $CO_2$ recovery column 3 via the $CO_2$ recovery column top $CO_2$ transfer piping 15. The recovered $CO_2$ is transferred to the buffer tank 1 and recycled in the reaction in the carbonate ester reactor 2.

The mixture recovered from the $CO_2$ recovery column 3 is transferred to a dehydrating agent separation column 4 via the $CO_2$ recovery column bottom liquid transfer piping 14. A mixture of 2-furonitrile and 2-furamide is recovered from the bottom of the dehydrating agent separation column 4 via a dehydrating agent separation column bottom liquid transfer piping 16, and PrOH and DPrC are recovered from the top of the dehydrating agent separation column 4 via a dehydrating agent separation column top liquid transfer piping 17.

The mixture recovered from the bottom of the dehydrating agent separation column 4 is transferred to the amide separation column 6 via the dehydrating agent separation column bottom liquid transfer piping 16. 2-Furamide (20) is recovered from the bottom of the amide separation column 6, and 2-furonitrile is recovered from the top of the amide separation column 6. The recovered 2-furonitrile is transferred to the buffer tank 1 via the amide separation column top

liquid transfer piping 21 and recycled in the reaction in the carbonate ester reactor 2.

PrOH and DPrC recovered from the top of the dehydrating agent separation column 4 is transferred to a carbonate ester recovery column 5 via the dehydrating agent separation column top liquid transfer piping 17. DPrC is recovered from the bottom of the carbonate ester recovery column 5 via a carbonate ester recovery column bottom liquid transfer piping 18, and PrOH is recovered from the top of the carbonate ester recovery column 5 via the carbonate ester recovery column top liquid transfer piping 19. The recovered PrOH is transferred to the buffer tank 1 and recycled in the reaction in the carbonate ester reactor 2.

(Second Reaction Step)

In the second reaction step, 2-furonitrile is produced by a dehydration reaction of 2-furamide in a nitrile regeneration gas phase reactor 8.

2-Furamide recovered from the amide separation column 6 is transferred to a vaporizer 7 via an amide separation column bottom liquid transfer piping 20. Preferably, it is mixed with an inert gas that is nitrogen or the like, the mixture is heated to a temperature near the boiling point of the amide compound to provide a gas or a mixed gas composed of gas and droplets, and it is transferred to the nitrile regeneration gas phase reactor 8 via a vaporizer-nitrile regeneration gas phase reactor connection piping 22. The form of the vaporizer 7 for vaporizing the amide compound, etc. is not particularly restricted, and any of an ejector type vaporizer, a contact type vaporizer, a bubbling device, and the like may be used.

In the apparatus for producing a nitrile compound to be used in the present invention (nitrile regeneration gas phase reactor 8), 2-furamide, and preferably, an inert gas that is nitrogen or the like, etc. are brought into contact with a catalyst containing a carried basic metal oxide in the gas phase to cause a dehydration reaction of 2-furamide. By this dehydration reaction, 2-furonitrile is produced. When transferring the amide compound, for the purpose of preventing the trouble of closure, the amide compound can be dissolved in a transfer solvent to transfer the liquid. When using an amide transfer solvent, it is preferable that the solvent is also vaporized together with amide to carry out a nitrile regeneration gas phase reaction. In this case, a solvent vapor can be used instead of the inert gas.

The form of the nitrile regeneration gas phase reactor 8 is not particularly restricted. It is preferable to use a gas phase reaction in which the amide compound in the form of gas or mist is allowed to flow through the catalyst layer together with the inert gas and the like, and a gas phase reaction apparatus with a fixed bed, fluidized bed, or the like can be applied thereto.

Thus, for efficiently promoting the dehydration reaction by bringing 2-furamide into contact with the catalyst, etc. in the gas phase, the above-described various reaction conditions related to the dehydration reaction or reaction conditions described in the Examples below are employed as appropriate.

The mixed gas containing 2-furonitrile is transferred from the gas phase reactor 8 to an $H_2O$ separation apparatus 9 via a gas phase reaction product transfer piping 23. Water and nitrogen gas are separated from the mixed gas in the $H_2O$ separation apparatus 9, and 2-furonitrile and 2-furamide recovered are transferred from the $H_2O$ separation apparatus 9 to the amide separation column 6 via an $H_2O$ separation apparatus high boiling transfer piping 24. Also, 2-furamide is recovered from the bottom of the amide separation column 6 via the amide separation column bottom liquid transfer piping 20 and transferred to the vaporizer 7, and 2-furonitrile is recovered from the top of the amide separation column 6. The recovered 2-furonitrile is transferred to the buffer tank 1 via the amide separation column top liquid transfer piping 21 and recycled in the reaction in the carbonate ester reactor 2.

Furthermore, nitrogen gas and water separated in the $H_2O$ separation apparatus 9 are transferred to an $N_2$ recovery apparatus 10 via an $H_2O$ separation apparatus light boiling transfer piping 25, and water is separated in the $N_2$ recovery apparatus 10 to recover nitrogen gas. Water separated in the $N_2$ recovery apparatus 10 is transferred to the outside of the carbonate ester apparatus via an $N_2$ recovery apparatus water transfer piping 26. Nitrogen gas recovered in the $N_2$ recovery apparatus 10 is transferred to the vaporizer 7 via an $N_2$ recovery apparatus $N_2$ transfer piping 27, and it can be used in the gas phase reaction. In the case where an amide compound transfer solvent is used, a step of recovering the solvent is provided separately, thereby reusing the solvent for transferring the amide compound. The form of each of the $H_2O$ separation apparatus 9 and the $N_2$ recovery apparatus 10 is not particularly restricted, and any of a cooling system, a membrane separation apparatus, and the like may be used.

As described above, in the present invention, dehydration of the amide compound can be promoted by the contact step in the gas phase, and in addition, the reaction product and compounds to be reused can be separated by distillation or the like without solid-liquid separation. For this reason, according to the present invention, it is possible to efficiently produce a carbonate ester in a smaller number of production steps while simplifying apparatuses.

## EXAMPLES

Hereinafter, the present invention will be more specifically described by way of examples, but the present invention is not limited thereto. Firstly, examples and comparative example of the method for producing furonitrile will be

described below.

Example 1 (example using vaporizer (heat medium) and reactor (mantle))

$SiO_2$ as a carrier (manufactured by Fuji Silysia Chemical Ltd., CARiACT, G-6 (carrier diameter: 0.075 to 0.150 mm)) was pre-baked at 700°C for about 1 hour. After that, for carrying Cs as an alkali metal, an aqueous solution was prepared using $CsNO_3$ (manufactured by Wako Pure Chemical Industries, Ltd.) such that the final amount of Cs metal to be carried would be 0.5 mmol/g, and $SiO_2$ was impregnated with the aqueous solution. After that, the obtained material was dried at 110°C for about 6 hours and then baked at 500°C for about 3 hours, thereby obtaining a $Cs_2O/SiO_2$ catalyst.

A reaction tube 36 made of SUS 316 having an inner diameter of 10.8 mm and a length of 35 cm was filled with the catalyst produced by the above-described production method (see Figure 1). Furthermore, immediately above the reaction tube 36, a vaporizing chamber 33 made of SUS 316 having an inner diameter of 10.8 mm and a length of 35 cm filled with Dixon packing was provided.

60 g of 2-furamide (2-FA) was heated and mixed with diphenyl ether serving as a transfer solvent (276 g) in an oil bath at 180°C, and the resulting mixed solution of 2-FA and diphenyl ether was put into a raw material container 31.

The vaporizing chamber 33 connected to the raw material container 31 was heated by a heat medium double pipe 34, and the reaction tube 36 was heated by a mantle heater 35 (see Figure 1). A piping at the exit side of the reaction tube 36 was kept at 180°C. For recovering a reaction product, dry ice/methanol cooling trap containers 37 and 38, and a trap container 39 were located. Also, a piping at the exit side of the trap container 39 was connected to a vacuum pump 40, and the pressure in the vaporizing chamber 33 and the reaction tube 36 was reduced to 40 (kPa).

After the temperature of the vaporizing chamber and the reaction tube became stable at 280°C, a plunger pump 32 was started, and a reaction was carried out for the time described in the column of the contact time with catalyst in Table 1 below.

Examples 2 and 3

Using the same reaction apparatus as in Example 1, the dehydration reaction of 2-furamide was carried out by the same method as in Example 1, except that the reaction conditions were changed as shown in Table 1 below. The trap containers in which the reaction product produced by the dehydration reaction was recovered are as follows.

Example 1: dry ice/methanol cooling trap containers 37 and 38, and trap container 39
Example 2: dry ice/methanol cooling trap containers 37 and 38, and trap container 39
Example 3: liquid nitrogen cooling trap container 7, dry ice/methanol cooling trap container 38, and trap container 39

Comparative Example 1 (comparative example by liquid phase reaction)

A $Cs_2O/SiO_2$ catalyst was produced by the same steps as in Example 1.

Next, a two-necked round-bottom flask was used as a reactor, and a magnetic stirring bar, the above-described $Cs_2O/SiO_2$ catalyst (0.2 g (Cs: 0.10 mmol)), 2-furamide (2-FA, 1.116 g (10 mmol), manufactured by Tokyo Chemical Industry Co., Ltd.), and diphenyl ether (37.4367 g (220 mmol), manufactured by Tokyo Chemical Industry Co., Ltd.) were introduced into the reactor.

Further, a thermometer and a first air cooling tube were attached to the reactor, a distilling head equipped with a thermometer was attached to the upper end of the first air cooling tube, and a second air cooling tube, a receiver, a trap, and a vacuum pump were connected to the distilling head to provide a reaction apparatus. The receiver and the trap were cooled using dry ice/methanol.

Subsequently, the pressure in the above-described reaction apparatus was reduced to 53.2 kPa (399 Torr) with the vacuum pump, the reaction solution was heated and maintained in a boiling state, and the reaction was carried out. The reaction was started when the reaction solution temperature reached 143°C, and the reaction was performed for 1.5 hours.

More detailed conditions for the above-described contact reaction (dehydration reaction) were as shown in Table 1. After the reaction was completed, the obtained reaction product was recovered and analyzed with GC-FID.

The analysis conditions for the results of the contact reaction (dehydration reaction) were as shown below.

[Analysis Conditions]

(GC-FID)

Shimadzu GC-2014, column: TC-17 (length: 30 m, inner diameter: 0.25 mmID, thickness of liquid phase: 0.25 μm)
Temperature of vaporizing chamber: 250°C, Detector: 250°C, Carrier $N_2$: 175 kPa, Flow rate of column: 10.3 mL/min, Split ratio: 5.0
Temperature program: [kept at 40°C for 10 min] → [elevated to 250°C, 12°C/min] → [kept at 250°C for 5 min]

[Yield of 2-furonitrile (mol/min)] = (Recovery amount of produced 2- furonitrile mol)/(Reaction time (min))

[Yield of 2-furonitrile (mol/day)] = (Recovery amount of produced 2-furonitrile mol)/(Reaction time (day))

[Yield of 2-furonitrile] = [Flow rate of produced 2-furonitrile (mol/min)]/[Flow rate of 2-furamide as raw material (mol/min)]

[Yield of furan as by-product] = [Flow rate of produced furan (mol/min)]/[Flow rate of 2-furamide as raw material (mol/min)]

[By-product ratio] = [Yield of furan as by-product]/[Yield of 2-furonitrile]

[Space velocity: SV ($hr^{-1}$)] = (Amount of gas passing through catalyst layer ($L\cdot hr^{-1}$))/(Amount of catalyst (L))

Amount of gas: the sum of volumes of gases of nitrogen, 2-FA (2-furamide), and solvent ($L\cdot hr^{-1}$)
[Catalyst filling height (height of catalyst layer in reactor)]: height of catalyst filled in reaction tube (m)

[Contact time with catalyst (sec)] = [catalyst filling height (m)]/[linear velocity (linear velocity of gas in reactor) (m/sec)]

[Carrier Diameter of Catalyst]

The value of the carrier diameter of the catalyst is measured based on the sieving method: Test sieving - General requirements defined in JIS Z 8815.

[Temperature of Vaporizing Chamber and Reaction Tube, and Reaction Temperature (Contact Temperature)]

The reaction temperature in the dehydration reaction was measured by inserting a sheath tube made of SUS 316 with a built-in thermometer from the lower portion of a reaction tube, which is illustrated as reaction tube 6 in the schematic diagram of the reaction apparatus shown in Figure 1, to a position immediately below the catalyst layer. That is, the temperature immediately below the catalyst layer was measured, and the temperature before allowing the raw materials to flow was defined as the temperature of the vaporizing chamber and the reaction tube, whereas the temperature after allowing the raw materials to flow was defined as the reaction temperature (contact temperature).

[Detection of Unknown Component (UK Detection)]

In the case where a compound (unknown component) whose molecular structure could not be determined was detected by the measurement method for the product of the contact reaction (dehydration reaction) described in the above-described column of [Analysis Conditions], the result was designated as "Present", and in the case where no unknown component was detected, the result was designated as "Absent".

[Reaction Pressure (Differential Pressure)]

For example, as shown in Figure 2, digital pressure gauges were attached near the inlet of the reactor, that is, near the inlet of the vaporizing chamber 33 [A: pressure at upper portion of vaporizer], and near the outlet of the reactor, that is, near the outlet of the reaction tube 36 [B: pressure at lower portion of catalyst layer], and the pressure was measured.
In Table 1, the reaction pressure (differential pressure) is shown as the difference between the measured pressures [A - B] (kPa), and the reaction pressure is shown as the average of the inlet and outlet pressures [(A + B)/2] (kPa).

The pressure values are the average values (kPa) of the respective sampling data.

[Gas volume velocity (V = (nRT)/P)]

The gas volume velocity (V) was calculated according to the following expression.

$$V = (nRT)/P$$

n: total amount of substances of nitrogen, 2-FA (2-furamide), and solvent (mol)
R: gas constant
T: reaction temperature (K: temperature immediately below catalyst layer)
P: reaction pressure (Pa)
Note that the gas volume velocity (V) was measured in order to calculate the contact time with catalyst (sec), and the value of the contact time with catalyst [sec] is calculated according to the above-described expression, i.e., [catalyst filling height (m)]/[linear velocity (linear velocity of gas in reactor) (m/sec)],
but it can also be determined by the expression: contact time with catalyst [sec] = catalyst volume [$m^3$] ÷ gas volume velocity [$m^3$/sec].

[Table 1]

| | | Example 1 | Example 2 | Example 3 | Comparative Example 1 |
|---|---|---|---|---|---|
| Desired product | | 2-Furonitrile (2-FN) | 2-Furonitrile (2-FN) | 2-Furonitrile (2-FN) | 2-Furonitrile (2-FN) |
| Type of diluting agent | | Phenyl ether | Phenyl ether | Phenyl ether | Phenyl ether |
| Reaction apparatus | | Vaporizer (heat medium) Reactor (mantle) | Vaporizer (heat medium) Reactor (mantle) | Vaporizer (heat medium) Reactor (mantle) | Eggplant flask, stirring bar, air cooling tube, receiver |
| Capturing method (cold trap) | | 1. Dry ice/ methanol 2. Dry ice/ methanol | 1. Dry ice/ methanol 2. Dry ice/ methanol | 1. Dry ice/ methanol 2. Liquid nitrogen | Dry ice/ methanol |
| Inner diameter of reactor | mm | 9.4 | 9.4 | 9.4 | - |
| Cross section of reactor | $mm^2$ | 69.4 | 69.4 | 69.4 | - |
| Catalyst | | $Cs_2O/SiO_2$(G6) | $Cs_2O/SiO_2$(G6) | $Cs_2O/SiO_2$(G6) | $Cs_2O/SiO_2$(G6) |
| Amount of catalyst ($Cs_2O/SiO_2$) | g | 1.0 | 1.0 | 1.0 | 0.2 |
| Height of catalyst | mm | 24.4 | 24.4 | 24.4 | - |
| Catalyst filling volume | $mm^3$ | 1695 | 1695 | 1695 | - |
| Vaporizing chamber (heat medium) | °C | 280 | 300 | 300 | - |
| Reactor (mantle) | °C | 280 | 300 | 300 | - |
| Reaction temperature (immediately below catalyst layer) | °C | 270.6 | 289.7 | 289.0 | - |
| Block heater heating temperature | °C | - | - | - | 246.0 |
| Reaction temperature (reaction solution temperature) | °C | - | - | - | 221.0 |
| Pressure at upper portion of vaporizer | kPa | 46.7 | 52.3 | 49.1 | - |
| Pressure at lower portion of catalyst layer | kPa | 39.9 | 40 | 37.875 | - |
| Reaction pressure | kPa | 43.3 | 46.2 | 43.5 | 53.2 |
| Differential pressure | kPa | 6.8 | 12.3 | 11.2 | - |
| Gas volume velocity (V = (nRT)/P) | $m^3$/min | 0.00081 | 0.00136 | 0.00148 | - |
| Flow rate of diluting agent | mol/min | 0.007 | 0.01 | 0.01 | - |
| Flow rate of 2-furamide | mol/min | 0.001 | 0.003 | 0.003 | - |
| Amount of diluting agent charged | mL | - | - | - | 40 |
| Amount of diluting agent charged | g | - | - | - | 37.42 |
| Amount of diluting agent charged | mol | - | - | - | 0.220 |
| Amount of 2-furamide charged | mol | - | - | - | 0.010 |
| Diluting agent/2-furamide (molar ratio) | | 9.00 | 3.00 | 3.00 | 22.10 |
| Flow rate of raw material | mol/min | 0.00777 | 0.01346 | 0.01374 | - |
| Raw material 2-furamide mol% | % | 10.0 | 25.0 | 25.0 | - |
| Linear velocity | m/sec | 0.1948 | 0.3275 | 0.3546 | - |
| Contact time | sec | 0.125 | 0.075 | 0.069 | - |
| Conversion rate of 2-furamide | % | 61.7 | 48.0 | 45.2 | 21.5 |
| Yield of 2-furonitrile | % | 61.6 | 47.9 | 45.1 | 16.6 |
| Yield of 2-furonitrile | mol/min | 0.000478 | 0.001611 | 0.001549 | - |
| Yield of 2-furonitrile | mol/day | 0.69 | 2.32 | 2.23 | - |
| By-product - yield (furan) | % | 0.09 | 0.08 | 0.06 | 0.065 |
| By-product - by-product ratio (furan) | % | 0.14 | 0.17 | 0.12 | 0.391 |
| Presence or absence of UK detection | | Absent | Absent | Absent | Absent |
| Reaction time | | 0.125 sec | 0.075 sec | 0.069 sec | 90 min |

As is apparent from the results of Examples and Comparative Example, in the dehydration reaction in the gas phase in Examples, a high yield of 2-furonitrile was realized despite the very short reaction time, as compared to the dehydration reaction in the liquid phase in Comparative Example. That is, although the yield of 2-furonitrile in Comparative Example was 21%, the yield in Examples was 45 to 62%. Also, in the dehydration reaction in the gas phase in Examples, the by-product ratio of furan as a by-product was suppressed.

With reference to the attached drawings, the suitable embodiments of the present invention are described in detail above, but the present invention is not limited to the examples. Those skilled in the art of the present invention would obviously conceive any of various altered or modified examples within the scope of the technical idea recited in the claims, and it is understood that such altered or modified examples are duly encompassed in the technical scope of the present

invention.

DESCRIPTION OF REFERENCE NUMERALS

- 31 raw material container
- 32 plunger pump
- 33 vaporizing chamber
- 34 heat medium double pipe
- 35 mantle heater
- 36 reaction tube
- 37, 38 cooling trap container
- 39 trap container
- 40 vacuum pump

## Claims

1. A method for producing a furonitrile compound, the method comprising a dehydration reaction represented by the following formula (1), in which a furamide compound is dehydrated,
wherein the dehydration reaction has a contact step in which the furamide compound and a diluting agent are brought into contact with a catalyst in a gas phase:

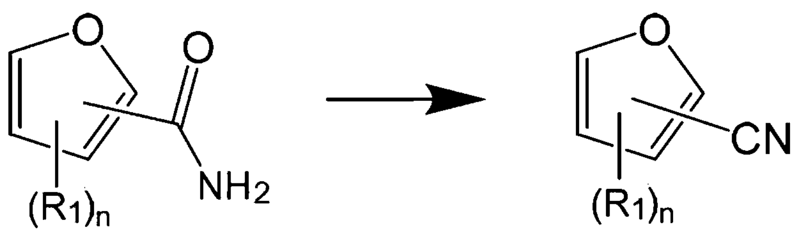

(1)

in formula (1),

$R_1$ represents a hydrogen atom, a halogen atom, an alkyl group having 1 to 20 carbon atoms and optionally having a substituent, an alkoxyl group having 1 to 20 carbon atoms and optionally having a substituent, a cycloalkyl group having 6 to 20 carbon atoms and optionally having a substituent, an aryl group having 6 to 20 carbon atoms and optionally having a substituent, a cycloalkoxyl group having 6 to 20 carbon atoms and optionally having a substituent, an aryloxy group having 6 to 20 carbon atoms and optionally having a substituent, or a cyclic compound group of a 3- to 20-membered ring containing at least two types of different elements and optionally having a substituent; and
n represents an integer of 0 to 3.

2. The method for producing a furonitrile compound according to claim 1, wherein $R_1$ is a hydrogen atom, an alkyl group having 1 to 20 carbon atoms, a cycloalkyl group having 6 to 20 carbon atoms, or an aryl group having 6 to 20 carbon atoms.

3. The method for producing a furonitrile compound according to claim 1, wherein a temperature at which the furamide compound and the diluting agent are brought into contact with the catalyst in the contact step is 150°C or higher.

4. The method for producing a furonitrile compound according to claim 1, wherein a pressure in a reaction system in the contact step is 101.3 kPa or lower.

5. The method for producing a furonitrile compound according to claim 1, wherein a contact time of the furamide compound with the catalyst in the gas phase is 0.001 seconds or longer and shorter than 10 seconds.

6. The method for producing a furonitrile compound according to claim 1, wherein the catalyst comprises an alkali metal.

7. The method for producing a furonitrile compound according to claim 1, wherein the diluting agent comprises at least any of a solvent and an inert gas.

8. The method for producing a furonitrile compound according to claim 7, wherein the solvent comprises at least an aromatic compound.

9. The method for producing a furonitrile compound according to claim 8, wherein the aromatic compound is represented by the following formula (2):

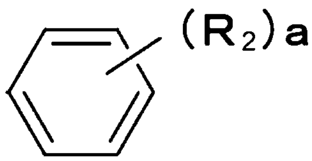

(2)

wherein

$R_2$ is each independently selected from an alkyl group having 1 to 10 carbon atoms and optionally having a substituent, an alkoxy group having 1 to 10 carbon atoms and optionally having a substituent, an aryl group having 6 to 30 carbon atoms and optionally having a substituent, and an aryl group having 6 to 30 carbon atoms and optionally having a substituent; and
a is an integer of 1 to 6.

10. The method for producing a furonitrile compound according to claim 8, wherein the aromatic compound has one or two aromatic rings and one or more selected from an alkyl group, an alkoxy group, and an aryloxy group.

11. The method for producing a furonitrile compound according to claim 8, wherein the aromatic compound comprises at least any of phenyl ether, 1,3,5-trimethylbenzene, p-cymene, and anisole.

12. The method for producing a furonitrile compound according to claim 7, wherein the solvent is compatible with the furamide compound.

13. The method for producing a furonitrile compound according to claim 7, wherein an inert gas in a vaporized state is used in the contact step.

14. The method for producing a furonitrile compound according to claim 7, wherein the inert gas comprises at least nitrogen gas.

15. A method for producing a carbonate ester, the method comprising:

a first reaction step that comprises a carbonate ester production reaction in which an alcohol is allowed to react with carbon dioxide in the presence of an furonitrile compound to produce a carbonate ester and water, and a hydration reaction in which the furonitrile compound is hydrated with the produced water to produce a furamide compound; and
a second reaction step in which the furamide compound is separated from a reaction system of the first reaction step and then the furamide compound is regenerated into a furonitrile compound by means of a dehydration reaction in which the furamide compound is dehydrated, the dehydration reaction having the contact step according to any one of claims 1 to 14,
wherein at least a part of the furonitrile compound regenerated in the second reaction step is used in the first reaction step.

16. The method for producing a carbonate ester according to claim 15, wherein a catalyst comprising cerium oxide is used in the carbonate ester production reaction.

17. The method for producing a carbonate ester according to claim 15, wherein the alcohol comprises an alcohol having 1

to 6 carbon atoms.

Figure 1

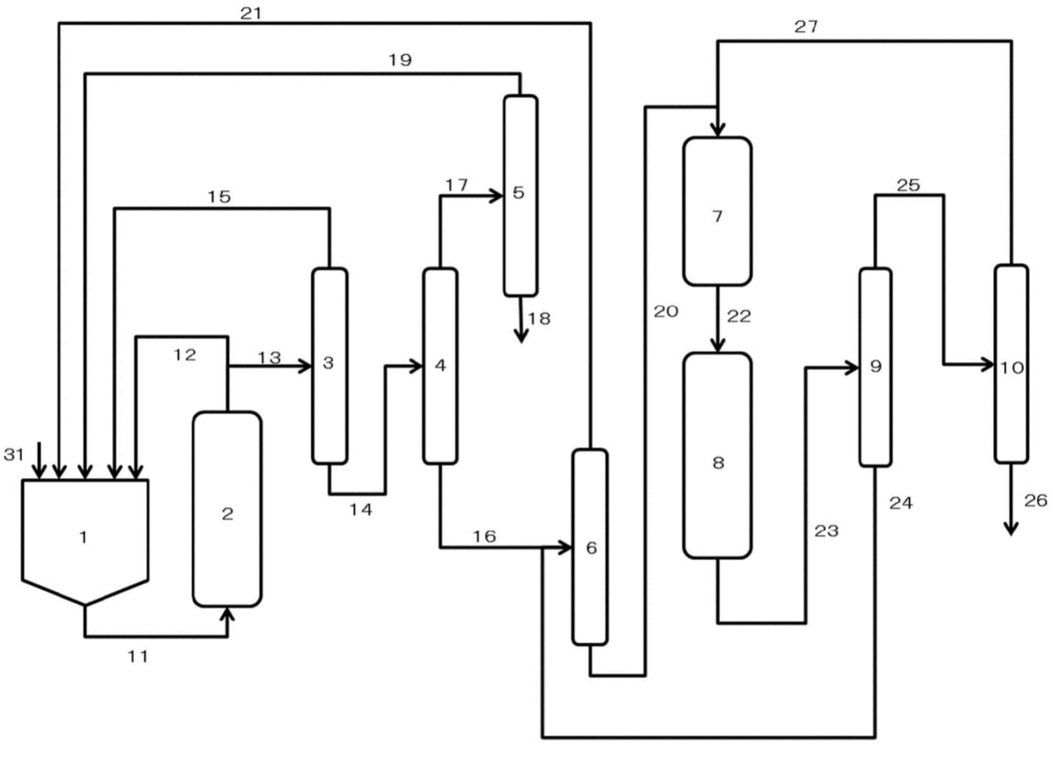

Figure 2

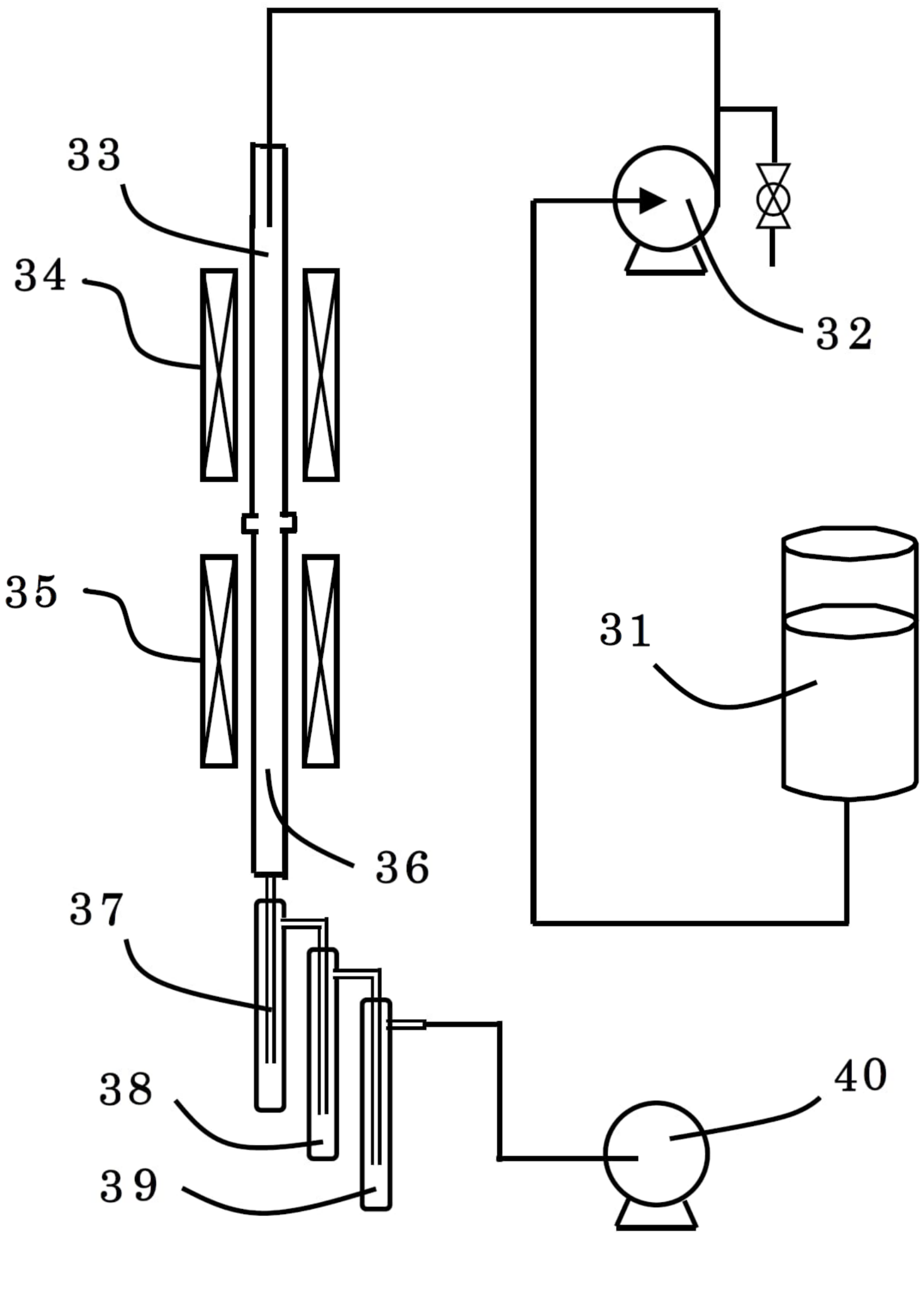

## INTERNATIONAL SEARCH REPORT

International application No. **PCT/JP2024/024148**

### A. CLASSIFICATION OF SUBJECT MATTER

***C07D 307/68***(2006.01)i; ***C07B 61/00***(2006.01)i
FI: C07D307/68; C07B61/00 300

According to International Patent Classification (IPC) or to both national classification and IPC

### B. FIELDS SEARCHED

Minimum documentation searched (classification system followed by classification symbols)

C07D307/68; C07B61/00

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

CAplus/REGISTRY (STN)

### C. DOCUMENTS CONSIDERED TO BE RELEVANT

| Category* | Citation of document, with indication, where appropriate, of the relevant passages | Relevant to claim No. |
|---|---|---|
| Y | WO 2022/181670 A1 (MITSUBISHI GAS CHEMICAL COMPANY, INC.) 01 September 2022 (2022-09-01) claims, examples | 1-17 |
| Y | CN 112495362 A (SHANXI INST COAL CHEMISTRY CAS) 16 March 2021 (2021-03-16) claims, examples | 1-17 |
| A | CAMPBELL, Jacqueline A. et al., Laboratory-Scale Synthesis of Nitriles by Catalysed Dehydration of Amides and Oximes under Flash Vacuum Pyrolysis (FVP) Conditions, Synthesis, 2007, no. 20, pp. 3179-3184 table 1 | 1-17 |

☐ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **10 September 2024** | **24 September 2024** |
| Name and mailing address of the ISA/JP<br>**Japan Patent Office (ISA/JP)**<br>**3-4-3 Kasumigaseki, Chiyoda-ku, Tokyo 100-8915**<br>**Japan** | Authorized officer<br><br>Telephone No. |

Form PCT/ISA/210 (second sheet) (July 2022)

**INTERNATIONAL SEARCH REPORT**
**Information on patent family members**

International application No.
**PCT/JP2024/024148**

| Patent document cited in search report | Publication date (day/month/year) | Patent family member(s) | Publication date (day/month/year) |
|---|---|---|---|
| WO 2022/181670 A1 | 01 September 2022 | EP 4299567 A1<br>claims, examples<br>CN 116867772 A<br>KR 10-2023-0151985 A<br>TW 202302552 A | |
| CN 112495362 A | 16 March 2021 | (Family: none) | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2015099053 A
- WO 2020022416 A
- WO 2022181670 A